# FASCICULE DE BREVET EUROPEEN

(11) **EP 2 790 706 B1**
(45) Date de publication et mention de la délivrance du brevet: **08.05.2019**
(21) Numéro de dépôt: 12813926.8
(22) Date de dépôt: 13.12.2012
(51) Int. Cl.: A61K 31/575, A61K 36/21, A61P 21/00

(54) **PHYTOECDYSONES POUR LEUR UTILISATION DANS L'AMÉLIORATION DE LA QUALITÉ MUSCULAIRE DE MAMMIFÈRES OBÈSES ET SARCOPÉNIQUES**
PHYTOECDYSONE ZUR VERWENDUNG ZUR VERBESSERUNG VON MUSKULÄRER QUALITÄT BEI ÜBERGEWICHTIGEN UND SARKOPENISCHEN SAUGETIEREN
PHYTOECDYSONES FOR USE IN AMELIORATING THE MUSCULAR QUALITY OF OBESE AND SARCOPENIC MAMMALS

(30) Priorité: 13.12.2011 FR 1161519
(43) Date de publication de la demande: 22.10.2014
(73) Titulaire: Biophytis, 75001 Paris (FR); Sorbonne Université, 75006 Paris (FR); Institut National De La Recherche Agronomique, 75007 Paris (FR)
(72) Inventeur: VEILLET, Stanislas, 91600 Savigny sur Orge (FR); LAFONT, René, F-75016 Paris (FR); FOUCAULT, Anne-Sophie, F-75013 Paris (FR); DIOH, Waly, F-91220 Bretigny sur Orge (FR); QUIGNARD-BOULANGÉ, Annie, F-75116 Paris (FR)
(74) Mandataire: Ipside
(86) Numéro de dépôt international: PCT/FR2012/052931
(87) Numéro de publication internationale: WO 2013/088084

(56) Documents cités:
- WO-A2-2010/040345
- DE-A1-102009 011 264
- FR-A1- 2 924 346
- GORELICK-FELDMAN J ET AL: "Ecdysteroids elicit a rapid Ca<2+> flux leading to Akt activation and increased protein synthesis in skeletal muscle cells", STEROIDS, ELSEVIER SCIENCE PUBLISHERS, NEW YORK, NY, US, vol. 75, no. 10, 1 octobre 2010 (2010-10-01), pages 632-637, XP027091741, ISSN: 0039-128X [extrait le 2010-06-16]
- DATABASE BIOSIS [Online] BIOSCIENCES INFORMATION SERVICE, PHILADELPHIA, PA, US; mars 2009 (2009-03), KIZELSZTEIN PABLO ET AL: "20-Hydroxyecdysone decreases weight and hyperglycemia in a diet-induced obesity mice model", XP002677354, Database accession no. PREV200900194812 & AMERICAN JOURNAL OF PHYSIOLOGY - ENDOCRINOLOGY AND METABOLISM, vol. 296, no. 3, mars 2009 (2009-03), pages E433-E439, ISSN: 0193-1849, DOI: DOI:10.1152/AJPENDO.90772.2008
- TOTH N ET AL: "20-Hydroxyecdysone increases fiber size in a muscle-specific fashion in rat", PHYTOMEDICINE, GUSTAV FISCHER VERLAG, STUTTGART, DE, vol. 15, no. 9, 3 septembre 2008 (2008-09-03), pages 691-698, XP023612056, ISSN: 0944-7113, DOI: 10.1016/J.PHYMED.2008.04.015 [extrait le 2008-06-26]

## Description

### Domaine technique

L'invention concerne les phytoecdysones, apportées pures ou sous forme d'extrait, pour leur utilisation dans l'amélioration de la qualité musculaire chez les mammifères.

L'invention permet également d'améliorer la qualité musculaire des mammifères sarcopéniques ainsi que des mammifères obèses soumis à un régime amaigrissant.

### Etat de la technique

La qualité musculaire peut se définir en premier lieu par la force musculaire, qui est liée à la masse ainsi qu'à la composition protéique et lipidique des muscles. La qualité musculaire des mammifères obèses est modifiée, en raison d'un excès d'apport de lipides dans le muscle (Magnusson et al., 2008). Les lipides non métabolisés s'accumulent dans les fibres musculaires (Goodpaster et al., 2000 ; Galgani et al., 2008), entraînant une modification du métabolisme musculaire, en particulier une diminution de la synthèse protéique (Anderson et al., 2008 ; Sitnick et al., 2009) et de l'activité des mitochondries (Kelley et al., 2002).

Une méthode pour qu'un mammifère obèse perde du poids et de la masse grasse est de suivre un régime hypocalorique. Ces régimes entraînent toutefois une perte importante de masse et de force musculaires (Bopp et al., 2008).

De même, le vieillissement entraîne une perte pathologique de masse et de force musculaires, pouvant conduire à une perte de mobilité anormale et un risque accru de chute (Boirie, 2008, 2009 ; Zamboni et al., 2008 ; Chérin, 2009 ; Rolland et Vellas, 2009 ; Pahor et al., 2009). La sarcopénie est le phénomène physiologique associé au vieillissement, par lequel un individu perd de la masse musculaire au profit de sa masse adipeuse.

Le phénomène de sarcopénie peut conduire à des cas particuliers d'obésité, connus sous le nom d'obésité sarcopénique.

La découverte de produits nutraceutiques ou pharmaceutiques permettant de limiter la perte de qualité musculaire chez des mammifères obèses, sarcopéniques ou obèses sarcopéniques, dans le cadre d'une intervention nutritionnelle, est donc un objectif poursuivi par de nombreux laboratoires et industriels, afin d'améliorer la prise en charge des mammifère obèses, sarcopénique ou obèses sarcopéniques par les nutritionnistes et cliniciens (Lynch, 2004 ; Bonnefoy, 2008 ; Chérin, 2009 ; Kim et al., 2010).

Les phytoecdysones sont des ecdystéroïdes d'origine végétale. Il s'agit de molécules naturelles de la famille des triterpènes, relativement abondantes dans le règne végétal où elles sont présentes chez 5% des plantes sauvages (Báthori et Pongrács, 2005). L'usage thérapeutique des dérivés d'ecdysones a en parti été exploré dans la demande internationale publiée sous le numéro WO2010040345A2.

Comme décrit dans le brevet FR2924346 au nom de la Demanderesse, les phytoecdysones, en particulier la 20-hydroxyecdysone, sont connues pour diminuer l'augmentation de la masse grasse chez les mammifères soumis à un régime obésifiant.

Une étude sur culture de cellules musculaires (Gorelick-Feldman al., 2010) a analysé les effets de la 20-hydroxyecdysone sur la réponse intracellulaire (production accrue de protéine), les voies de signalisations (taux de Ca2+, activation de Akt) et les inhibiteurs de 20-hydroxyecdysone (GPCR, PLC, PI3K).

Une étude de l'usage de la 20-hydroxyecdysone in vivo (Tóth et al, 2008) a montré chez le rat un effet d'augmentation de la taille des fibres du muscle soléaire en phase de régénération.

En outre, les phytoecdysones présentent des propriétés antioxydantes (Kuzmenko et al., 2001) et sont dépourvues de toxicité.

### Exposé de l'invention

Les inventeurs ont découvert que l'ingestion régulière ou non de phytoecdysones permet d'améliorer la qualité et/ou la force musculaire chez les mammifères atteint de sarcopénie et/ou atteints d'obésité sarcopénique.

Par amélioration de la qualité et/ou la force musculaire, on entend par exemple que l'ingestion de phytoecdysones, en particulier de 20-hydroxyecdysone, permet d'augmenter la masse maigre chez le mammifère soumis à un régime obésifiant, ainsi que la teneur en protéines du muscle dudit mammifère. Par ailleurs, l'ingestion de phytoecdysones permet de diminuer la perte de masse maigre chez le mammifère soumis à un régime amaigrissant. Enfin, la force musculaire dudit mammifère soumis à un régime amaigrissant, évaluée par l'intermédiaire d'un test, est également préservée par l'ingestion de phytoecdysones.

Un individu est considéré comme obèse dès lors que son indice de masse corporel (IMC) est supérieur à 30. Dans les cas d'obésité sarcopénique, l'IMC peut être inférieur à 30 en raison de la perte de masse musculaire au profit de la masse grasse.

L'invention propose donc de mettre en oeuvre des phytoecdysones, particulièrement la 20-hydroxyecdysone, pour améliorer ou maintenir la force musculaire chez les mammifères sarcopéniques.

Une forme particulière de l'invention met en oeuvre les phytoecdysones pour maintenir la force musculaire chez les mammifères obèses sarcopéniques

Une forme particulière de l'invention met en oeuvre les phytoecdysones pour diminuer la teneur en lipides et/ou augmenter la teneur en protéines des muscles des mammifères obèses sarcopéniques.

Une forme particulière de l'invention met en oeuvre les phytoecdysones pour maintenir la force musculaire chez les mammifères obèses soumis à un régime hypocalorique amaigrissant.

Les phytoecdysones utilisées peuvent être obtenues par extraction, à partir de plantes contenant des phytoecdysones. Les phytoecdysones utilisées peuvent également être des phytoecdysones de synthèse.

Les phytoecdysones sont préférentiellement choisies parmi la 20-hydroxyecdysone, la makisterone A, la 24-epi-makisterone A, la 24(28)-dehydro-makisterone A, la 20,26-dihydroxyecdysone et les mélanges de deux ou plus de ces composés.

Dans une forme particulière de l'invention la phytoecdysone choisie est préférentiellement la 20-hydroxyecdysone.

Les phytoecdysones peuvent être apportées pures, ou sous forme d'extrait de plantes plus ou moins enrichi. Avantageusement, les phytoecdysones mises en oeuvre selon l'invention se présentent sous la forme d'un extrait de plantes enrichi en phytoecdysones, ledit extrait contenant au moins 1% en masse de phytoecdysones. Préférentiellement, l'extrait contient entre 1% et 7% de phytoecdysones, plus préférentiellement entre 1.5% et 3% et encore plus préférentiellement 2% en masse de phytoecdysones.

Les plantes à partir desquelles sont réalisés les extraits selon l'invention sont avantageusement choisies parmi le quinoa, les épinards et les champignons.

Préférentiellement, l'extrait de plantes enrichi en phytoecdysones selon l'invention provient d'un extrait de quinoa. En effet, le quinoa est une pseudo céréale comestible naturellement riche en phytoecdysones (Zhu et al., 2001 ; Dini et al., 2005). Il est ainsi possible de compléter l'alimentation par ingestion d'extrait de quinoa enrichi en phytoecdysones, en introduisant cet extrait dans un aliment, tel qu'un produit laitier ou une boisson, ou en le consommant, en tant que complément alimentaire, par exemple sous forme de gélules.

Le quinoa représente à ce jour la plante alimentaire de loin la plus riche en phytoecdysones. Les graines de quinoa contiennent un mélange de phytoecdysones (Zhu et al., 2001). Ces phytoecdysones sont particulièrement abondantes dans l'enveloppe des graines de quinoa. Par exemple, une ration de graines de quinoa de 60 grammes (poids sec) comprend entre 15 et 20 milligrammes de 20-hydroxyecdysone.

L'épinard et certains champignons peuvent également être avantageusement utilisés pour la fabrication d'un extrait de plantes riche en phytoecdysones (Findeisen E, 2004).

Les phytoecdysones mises en oeuvre selon l'invention se présentent avantageusement sous la forme d'une composition pouvant être administrée par voie orale.

La composition s'entend par exemple d'un produit alimentaire tel qu'une boisson, un produit laitier ou autre. Bien entendu, la composition peut être une composition médicinale par exemple utilisée sous forme de pilules qui contiennent ainsi une dose bien précise de phytoecdysones.

### Brève description des figures

- Figure 1 : Graphique représentant le poids de carcasses de quatre groupes de souris selon un premier protocole expérimental ;
- Figure 2 : Graphique représentant la teneur en triglycérides du muscle quadriceps en fonction du régime et du traitement auxquels sont soumises les souris du premier protocole ;
- Figure 3 : Graphique représentant la teneur en protéines du muscle quadriceps en fonction du régime et du traitement auxquels auxquels sont soumises les souris du premier protocole ;
- Figure 4 : Graphique représentant les niveaux d'expression de gènes dans le muscle quadriceps en fonction du régime et du traitement auxquels auxquels sont soumises les souris du premier protocole ;
- Figure 5 : Consommation moyenne de nourriture (kcal/jour) des souris selon les différents traitements du premier protocole ;
- Figure 6 : Dépense énergétique moyenne (Watt) des souris selon les différents traitements du premier protocole ;.
- Figure 7 : Evolution de la masse maigre chez des sujets obèses supplémentés en extrait de quinoa (A) ou placebo (B) après une phase de régime hypocalorique de 6 semaines, selon un deuxième protocole expérimental ;
- Figure 8 : Evolution de la force mesurée avec le « grip test » chez des sujets obèses supplémentés en extrait de quinoa (A) ou placebo (B) après une phase de régime hypocalorique de 6 semaines selon le deuxième protocole ;
- Figure 9 : Formules chimiques de phytoecdysones présentes dans une composition selon un mode de réalisation de l'invention.

### Description détaillée

Dans l'invention, on propose d'apporter une dose concentrée de phytoecdysones pures, ou par le biais d'un extrait de plantes enrichi en phytoecdysones, afin d'améliorer l'état musculaire de personnes obèses, sarcopéniques, ou encore atteintes d'obésité sarcopénique.

Selon l'invention, il est possible d'apporter cette dose de phytoecdysones sous la forme d'un extrait de plantes, tel que de quinoa, incorporé par exemple dans un aliment entrant dans l'alimentation courante d'un individu. En effet, dans 4 grammes d'extrait de quinoa enrichi à hauteur de 0,5% en poids de phytoecdysones, on a 20 milligrammes de phytoecdysones. Pour obtenir la même quantité de phytoecdysones à partir de graines de quinoa, il faudrait consommer de 50 à 100 grammes de graines de quinoa non traitées (Dini et al., 2005). L'extrait de quinoa selon l'invention peut ainsi contenir jusqu'à 50 fois plus de phytoecdysones que les graines de quinoa dont l'extrait est issu.

### I - Exemple de procédé de préparation d'extrait de quinoa enrichi en phytoecdysones (Extrait A)

On procède à une extraction séquentielle par l'eau, en ajoutant 500g de graines de quinoa à 2 L d'eau bouillante, et on maintient l'ensemble pendant 5 minutes à 80 °C. On élimine l'eau et on réalise une seconde extraction avec 2 L d'un mélange éthanol-eau (1 :1) sous agitation pendant 20 minutes à 80 °C.

Une telle extraction séquentielle permet de supprimer de l'extrait les saponines, abondantes dans les graines de quinoa (Muir et al., 2002), qui conféreraient un goût amer audit extrait.

L'extrait éthanolique est filtré sur Miracloth™, évaporé à sec et repris avec 400 mL d'éthanol absolu, ce qui laisse un résidu insoluble abondant.

La fraction éthanolique est filtrée ou centrifugée puis séchée.

L'analyse chromatographique (HPLC) montre que cet extrait contient 2 ± 0,2 % en poids de 20-hydroxyecdysone (20E).

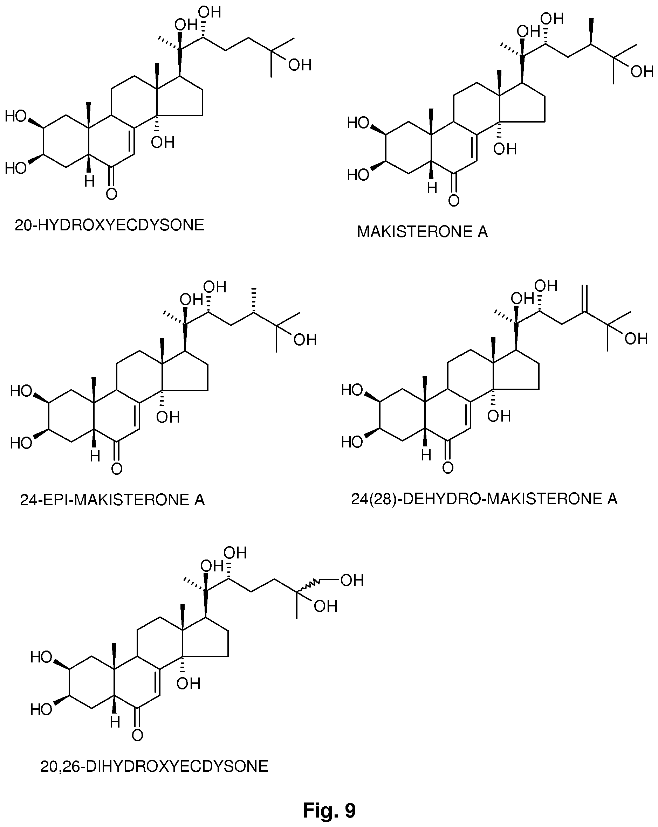

On obtient entre 150 et 200 milligrammes de phytoecdysones par kilogramme de graines de quinoa traitées, dont 85-90 % correspondent à la 20-hydroxyecdysone, et le restant à des ecdystéroïdes de structure très voisine comme la makisterone A, la 24-epi-makisterone A, la 24(28)-dehydro-makisterone A ou la 20,26-dihydroxyecdysone. Les structures de ces composés sont représentées à la figure 9.

Un extrait analogue à l'extrait A, utilisable dans le cadre de l'invention, est notamment commercialisé sous la marque Quinolia®.

### Il - Etude expérimentale de l'effet de la 20-hvdroxvecdvsone et de l'extrait A sur la composition musculaire de souris soumises à un régime hyperlipidique

### Protocole

On teste l'effet des phytoecdysones sur des souris soumises à un régime hyperlipidique pendant 3 semaines.

Le régime hyperlipidique HF consiste en l'apport de quantités importantes de matière grasse sous la forme de saindoux. Les souris choisies dans cette étude sont des souris C57BL/6J mâles, âgées de 6 semaines lorsqu'elles commencent l'expérimentation.

On teste également en parallèle des souris non soumises à un régime hyperlipidique constituant le régime normal témoin.

Les souris étudiées sont réparties en fonction des régimes et des traitements auxquels elles sont soumises : régime normal ou témoin (LF), régime hyperlipidique (HF), régime hyperlipidique complémenté en extrait A de quinoa (HFQ) et régime hyperlipidique complémenté en 20-hydroxyecdysone pure (HF20E).

Les souris sont soumises au régime tel que détaillé au tableau 1 ci-dessous, pendant trois semaines et les souris sous régime hyperlipidique sont traitées en parallèle avec la 20E pure ou par l'extrait A (2% de 20E). La concentration en 20E est ajustée à 40 mg par kg d'aliment.

Compte tenu de la quantité d'aliment ingérée en moyenne par les souris, la dose de 20E administrée correspond dans les 2 traitements à 5 mg de 20E par kg de poids corporel et par jour. L'aliment est fourni en excès, tous les jours, pour les deux régimes et les 3 traitements. En moyenne, 40 g d'aliment est apporté par cage et par jour, soit 6,5 g d'aliment par souris et par jour.

Le tableau 1 ci-dessous indique plus en détail la composition du régime alimentaire auquel les souris sont soumises :

**Tableau 1 : Composition des régimes**

| | Régime témoin LF | Régime hyperlipidique HF |
|---|---|---|
| | Composition (g/kg) | |
| Protéines de lait | 140 | 170 |
| Amidon | 622,4 | 360 |
| Saccharose | 100,3 | 57 |
| Huile de soja | 40 | 40 |
| Saindoux | 0 | 235* |
| Sels minéraux | 35 | 62,5 |
| Vitamines | 10 | 12,5 |
| Cellulose | 50 | 62,5 |
| Choline | 2,3 | 2,3 |

| | Energie (kcalories %) | |
|---|---|---|
| Protéines | 15 | 14 |
| Glucides | 76 | 35 |
| Lipides | 9 | 51 |

| | | |
|---|---|---|
| ** 56 % d'acides gras monoinsaturés, 29% d'acides gras saturés et 15% d'acides gras polyinsaturés (Ueda *et al,* 2011). | | |

### Résultats

### Mesure du poids des carcasses des animaux.

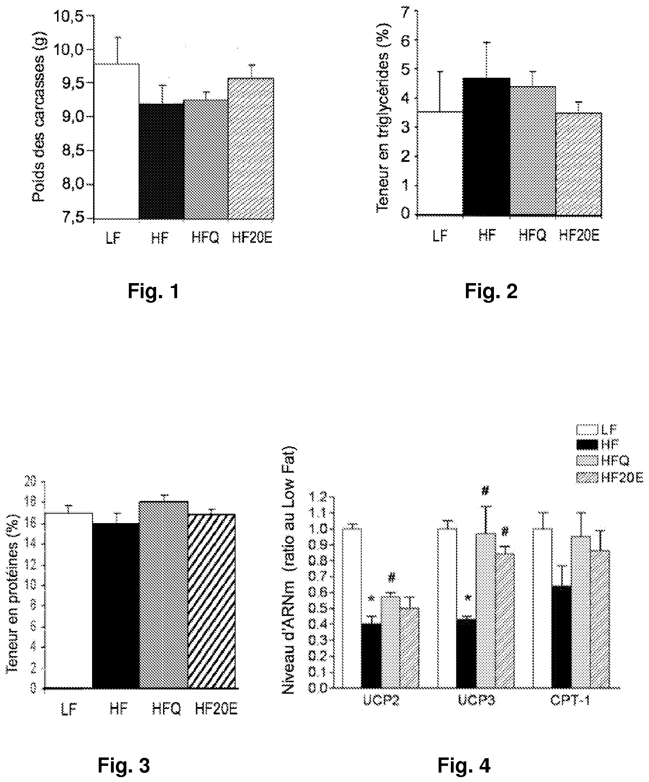

La figure 1 représente la masse maigre (carcasse dégraissée) des animaux en fin d'expérimentation. L'administration du régime gras entraîne une réduction du poids de carcasse de -5% par rapport au contrôle. Ce résultat est en accord avec la réduction des synthèses protéiques musculaires induites par un tel régime (Anderson et al., 2008). La supplémentation avec l'extrait A n'entraîne pas d'augmentation significative, mais la 20-hydroxyecdysone entraine une augmentation qui permet aux souris de retrouver quasiment le niveau du régime normal.

### Mesure de la teneur en triglycérides du muscle.

Au sacrifice, des aliquotes de muscle (quadriceps) sont prélevées pour être analysées. Sur la figure 2 est représenté un graphique montrant la teneur en triglycérides du muscle en fonction du régime et du traitement associé.

De manière attendue, on note une tendance à l'augmentation de la teneur en triglycérides du muscle plus importante chez les souris nourries avec le régime hyperlipidique que chez les souris témoins nourries avec le régime témoin (+30% d'augmentation).

Chez les souris ayant reçu un traitement, en association avec le régime hyperlipidique, les traitements avec la 20E pure et avec l'extrait A montrent une tendance à diminuer la teneur en triglycérides du muscle de 26% et 6% respectivement.

### Mesure de la teneur en protéines du muscle.

Sur la figure 3 est représenté un graphique montrant la teneur en protéines du muscle en fonction du régime et du traitement associé. Le régime hyperlipidique montre une tendance à diminuer (-5%) la teneur en protéines du muscle par rapport aux souris nourries avec le régime témoin.

Chez les souris ayant reçu un traitement, en association avec le régime hyperlipidique, les traitements avec la 20E pure et avec l'extrait A montrent une tendance à augmenter la teneur en protéines du muscle de 5% et 13%, respectivement, par rapport au traitement HF seul.

### Mesure de la quantité de transcrits de gènes dans le muscle.

Sur la figure 4 est représenté un graphique montrant la quantité de transcrits (ARNm) de gènes, mesurée dans le muscle, en fonction du régime et du traitement associé. Les quantités sont normalisées par rapport à la quantité mesurée dans le muscle des souris nourries avec le régime témoin.

Le régime hyperlipidique entraîne une forte diminution de la quantité de transcrits des gènes codant pour les protéines découplantes UCP2 et UCP3 par rapport à la quantité mesurée chez les souris nourries avec le régime témoin. Chez les souris ayant reçu un traitement, en association avec le régime hyperlipidique, le traitement avec la 20E pure se traduit par une augmentation de la quantité de transcrits du gène UCP3 et par une tendance à l'augmentation de la quantité de transcrits du gène UCP2. Chez les souris ayant reçu un traitement, en association avec le régime hyperlipidique, le traitement avec l'extrait A provoque une augmentation de la quantité de transcrits des gènes UCP2 et UCP3. Le régime hyperlipidique induit une diminution de la quantité de transcrits du gène codant pour le transporteur intracellulaire d'acides gras CPT-1 par rapport à la quantité mesurée chez les souris nourries avec le régime témoin. Les traitements avec la 20E pure et avec l'extrait A tendent donc à restaurer la quantité des transcrits à la valeur du régime témoin. Ces modifications sont en accord avec une amélioration de la capacité oxydative des muscles par le traitement avec la 20E pure et avec l'extrait A.

### Bilan énergétique

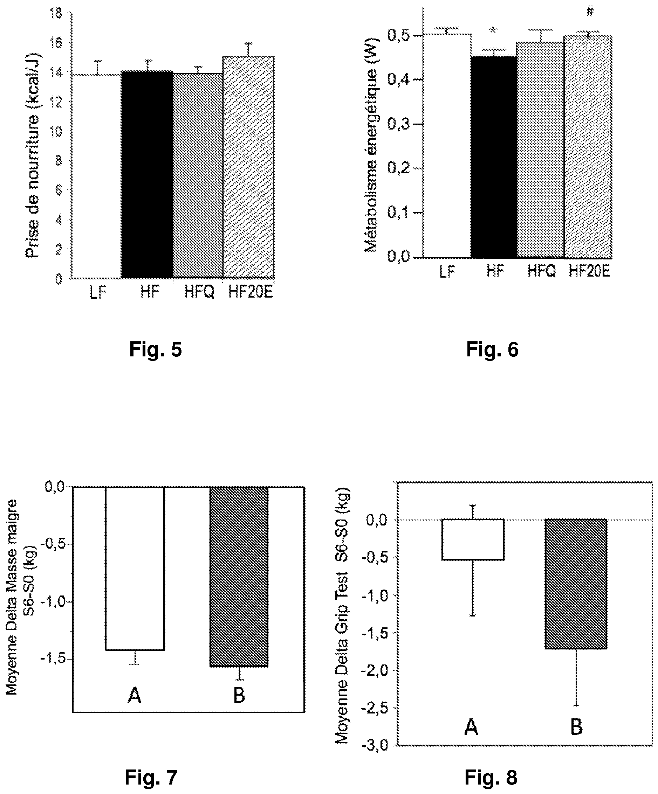

Les animaux nourris avec le régime hyperlipidique consomment une quantité d'aliment qui leur apporte la même quantité d'énergie (en kcal) que pour les animaux nourris avec le régime standard (figure 5). Il en va de même pour les animaux qui reçoivent l'extrait A ou la 20E pure. En revanche, la dépense énergétique de ces derniers est plus élevée (+ 9 %) que chez les animaux nourris avec le régime hyperlipidique seul (figure 6). Cette différence, bien que faible, a des conséquences importantes, car son effet est cumulatif au cours de la durée de l'expérimentation.

### Conclusion des expériences sur les souris

L'administration de la 20E pure, comme celle de l'extrait A, empêche le dépôt lipidique et la perte protéique dans le muscle induits par un régime hyperlipidique à base de saindoux. Ces deux traitements favorisent la métabolisation des acides gras, apportés en excès dans le muscle suite à l'administration du régime hyperlipidique.

L'augmentation de la dépense énergétique alors que les apports alimentaires restent constants peut expliquer les différences observées dans l'accumulation de masse grasse. Cette dépense énergétique accrue ne résulte pas d'une activité locomotrice accrue (mesurée dans les cages métaboliques) ; elle résulterait donc d'une augmentation de la thermogenèse.

### III - Etude clinique en double aveugle des effets de l'extrait A sur des individus obèses soumis à un régime hypocalorique pendant 6 semaines

### Protocole

L'effet de l'extrait A a été étudié sur la protection de la masse maigre lors d'un régime hypocalorique. Cet effet de l'extrait A a été étudié dans le cadre d'une étude clinique en double aveugle sur des sujets obèses soumis à un régime hypocalorique de 6 semaines. La protection de la masse maigre a été évaluée par la mesure de la force musculaire à l'aide d'un « grip test » et par l'estimation de la masse maigre lors d'une analyse de la composition corporelle par DEXA SCAN.

Les données de force musculaire et de masse maigre correspondent à des valeurs estimées. Pour tenir compte des différences dans la durée de la phase de régime hypocalorique, les données du grip test et de masse maigre ont d'abord été calculées par jour de durée effective, puis multipliées par 42 jours qui représentent la durée moyenne de la phase de régime hypocalorique des volontaires.

### Mesure de la perte de masse maigre lors du régime hypocalorique

L'effet de l'extrait A sur la protection de la masse maigre a été étudié lors d'un régime hypocalorique. Le produit confère une légère tendance à protéger la masse maigre par comparaison avec placebo (Figure 7).

Il est vraisemblable que les contraintes métaboliques liées à un régime fort surpassent tout le reste ; en effet, dans les études hors contexte de régime, les effets « anabolisants » de la 20-hydroxyecdysone sont nettement renforcés par un apport complémentaire de protéines (Simakin et al., 1988).

### Evolution du Grip test lors du régime hypocalorique

L'effet d'une administration de l'extrait A sur la qualité musculaire chez des sujets obèses soumis à un régime hypocalorique a été étudié. L'évolution mesurée lors du grip test après 6 semaines de régime (Figure 8) montre une meilleure protection de la force musculaire chez les sujets supplémentés en extrait A (-0,55 kg) que chez les sujets ayant reçu le placebo (-1,70 kg).

### Conclusions

L'administration de l'extrait A confère aux sujets obèses une meilleure protection de la masse maigre comme montré par l'analyse DEXA scan, avec une tendance à une moindre perte dans le cas de l'extrait A comparé au placebo. La qualité musculaire est aussi mieux protégée par l'administration de l'extrait A avec une perte moins importante comparée au groupe placebo.

### Références

Anderson SR, Gilge DA, Steiber AL, Prévis SF. 2008. Diet-induced obesity alters protein synthesis: tissue-specific effects in fasted versus fed mice. Metabolism 7(3): 347-54.
Chermnykh NS, Shimanovsky NL, Shutko GV, Syrov VN. 1988. Effects of methandrostenolone and ecdysterone on physical endurance of animais and protein metabolism in the skeletal muscles. Farmakologiya i Toksikologiya 6: 57-62.
Foucault AS, Lafont R, Dioh W, Fromentin G, Veillet S, Tomé D, Quignard-Boulangé A. 2008. Effets d'un extrait de quinoa enrichi en 20-hydroxyecdysone sur l'adiposité dans le cadre du syndrome métabolique. Nutrition Clinique et Métabolisme 22 (suppl.1), S103.
Gadzhieva RM, Portugalov SN, Paniushkin VV, Kondrat'eva II. 1995. A comparative study of the anabolic action of ecdysten, leveton and Prime Plus, préparations of plant origin. Eksp Klin Farmakologiya 58(5): 46-48.
Garcia-Martinez C, Sibille B, Solanes G, Darimont C, Mace K, Villarroya F, Gomez-Foix AM. 2001. Overexpression of UCP3 in cultured human muscle lowers mitochondrial membrane potential, raises ATP/ADP ratio, and favors fatty acid vs. glucose oxidation. FASEB J. 15: 2033-2035.
Gong DW, He Y, Karas M, Reitman M. 2000. Uncoupling protein-3 is a mediator of thermogenesis regulated by thyroid hormone, β3-adrenergic agonists, and leptin. J. Biol. Chem. 272(39): 24129-24132.
Gorelick-Feldman J, MacLean D, Ilic N, Poulev A, Lila MA, Raskin I. 2008. Phytoecdysteroids increase protein synthesis in skeletal muscle cells. J. Agric. Food Chem. 56: 3532-3537.
Gorelick-feldman J et al.: "Ecdysteroids elicit a rapid Ca 2+flux leading to Akt activation and increased protein synthesis in skeletal muscle cells", STEROIDS, ELSEVIER SCIENCE PUBLISHERS, NEW YORK, NY, US, vol. 75, no. 10, 1 octobre 2010 (2010-10-01), pages 632-637.
Li B, Nolte LA, Ju JS, et al. (2000) Skeletal muscle respiratory uncoupling prevents diet-induced obesity and insulin résistance in mice. Nature Medicine 6: 1115-1120.
Schrauwen P, Hesselink M (2002) Review. UCP2 and UCP3 in muscle controlling body metabolism. J. Exp. Biol. 205: 2275-2285.
Seidlova-Wuttke D, Ehrhardt C, Wuttke W. 2010. Metabolic effects of 20-OH-ecdysone in ovariectomized rats. J. Steroid Biochem. Mol. Biol. 119: 121-126.
Simakin SYu, Panyushkin VV, Portugalov SN, Kostina LV, Martisorov EG. 1988. Combined application of preparation Ecdysten and product Bodrost during training in cyclic sports. Sports Science Bulletin N°2, 29-31.
Tóth N, Szabó A, Kacsala P, Héger J, Zádor E. 2008. 20-Hydroxyecdysone increases fiber size in a muscle-specific fashion in rat. Phytomedicine 15: 691-698.
Ueda Y, Wang MF, Irei AV, Sarukura N, Sakai T, Hsu TF. 2011. Effect of Dietary Lipids on Longevity and Memory in the SAMP8 Mice. J. Nutr. Sci. Vitaminol. (Tokyo) 57(1): 36-41.
Veillet S, Lafont R. (2009) Use of phytoecdysones for the preparation of a composition for acting on metabolic syndrome. Patent WO 2009071804, application WO 2008-FR52088, demande FR 2007-59478 US 2011/0033561 A1.
Wang S, Subramaniam A, Cawthorne MA, Clapham JC (2003) Increased fatty acid oxidation in transgenic mice over-expressing UCP3 in skeletal muscle. Diabetes, Obesity and Metabolism 5: 295-301.

## Revendications

1. Phytoecdysones pour utilisation dans l'amélioration ou le maintien de la force musculaire chez les mammifères sarcopéniques.

2. Phytoecdysones selon la revendication 1, pour utilisation dans le maintien de la force musculaire chez les mammifères obèses sarcopéniques.

3. Phytoecdysones selon la revendication 2, pour utilisation dans la diminution de la teneur en lipides et/ou l'augmentation de la teneur en protéines des muscles des mammifères obèses sarcopéniques.

4. Phytoecdysones pour utilisation selon l'une des revendications 1 à 2, telles qu'elles consistent en la 20-hydroxyecdysone.

5. Phytoecdysones pour utilisation selon l'une des revendications précédentes, apportées sous forme d'un extrait de plantes enrichi en une ou plusieurs phytoecdysones.

6. Phytoecdysones pour utilisation selon la revendication 5, telles que l'extrait comporte au moins 1% en poids de phytoecdysones.

7. Phytoecdysones pour utilisation selon l'une des revendications 5 à 6, telles que l'extrait de plantes provient du quinoa.

8. Phytoecdysones pour utilisation selon l'une des revendications précédentes, incorporées à une composition apte à être administrée par voie orale.

## Patentansprüche

1. Phytoecdysone zur Verwendung in der Verbesserung oder dem Erhalt der Muskelkraft bei sarkopenischen Säugetieren.

2. Phytoecdysone nach Anspruch 1, zur Verwendung im Erhalt der Muskelkraft bei sarkopenisch-adipösen Säugetieren.

3. Phytoecdysone nach Anspruch 2, zur Verwendung in der Verringerung des Lipidgehalts und/oder der Steigerung des Proteingehalts der Muskeln von sarkopenisch-adipösen Säugetiere.

4. Phytoecdysone zur Verwendung nach einem der Ansprüche 1 bis 2, derart, dass sie in 20-Hydroxyecdyson bestehen.

5. Phytoecdysone zur Verwendung nach einem der vorstehenden Ansprüche, die in Form eines Pflanzenextraktes zugeführt werden, der an einem oder mehreren Phytoecdysonen angereichert ist.

6. Phytoecdysone zur Verwendung nach Anspruch 5, derart, dass der Extrakt mindestens 1Gewichts-% Phytoecdysone umfasst.

7. Phytoecdysone zur Verwendung nach einem der Ansprüche 5 bis 6, derart, dass der Pflanzenextrakt von Quinoa stammt.

8. Phytoecdysone zur Verwendung nach einem der vorstehenden Ansprüche, die in eine Zusammensetzung eingebunden sind, welche oral verabreicht werden kann.

## Claims

1. Phytoecdysones for use in improving or maintaining muscle strength of sarcopenic mammals.

2. Phytoecdysones according to claim 1, for use in maintaining muscle strength of obese sarcopenic mammals.

3. Phytoecdysones according to claim 2, for use in reducing the content of lipids and/or increasing the content of proteins in muscles of obese sarcopenic mammals.

4. Phytoecdysones for use according to one of claims 1 to 2, such that they consist of 20-hydroxyecdysone.

5. Phytoecdysones for use according to one of the preceding claims, supplied in the form of a plant extract enriched in one or several phytoecdysones.

6. Phytoecdysones for use according to claim 5, such that the extract comprises at least 1% by weight of phytoecdysones.

7. Phytoecdysones for use according to one of claims 5 to 6, such that the plant extract comes from quinoa.

8. Phytoecdysones for use according to one of the preceding claims, incorporated in a composition that can be administered orally.
